# EUROPEAN PATENT APPLICATION

(11) **EP 0 592 053 A1**
(43) Date of publication of application: **13.04.1994**
(21) Application number: 93202854.1
(22) Date of filing: 08.10.1993
(51) Int. Cl.: C08G 63/78, C07C 29/80

(54) **Method for recovering the tower bottom residues from the fractional distillation of ethylene glycol**

(30) Priority: 09.10.1992 IT MI922330
(71) Applicant: MONTEFIBRE S.p.A., I-20124 Milano (IT)
(72) Inventor: Vosa, Renato, I-81022 Casagiove (Caserta) (IT); Santagata, Salvatore, I-81030 Gricignano di Aversa (Caserta) (IT)
(74) Representative: Fusina, Gerolamo

(57) **Abstract**

Method for recovering the tower bottom residues produced by the fractional distillation of ethylene glycol, by polymerizing said residues, optionally diluted with ethylene glycol, with an aromatic dicarboxy acid and/or an ester thereof.

## Description

The present invention relates to a method for recovering the tower bottom residues produced by the fractional distillation of the excess of ethylene glycol resulting from the process for manufacturing an aromatic polyester based on such a glycol, and to the product obtained by starting from said residues.

The method according to the present invention is particularly directed to the recovery of materials which generally are waste byproducts destined to be burnt or disposed of into protected landfills.

Most processes for manufacturing ethylene glycol-based aromatic polyesters both by starting from aromatic diacids such as, e.g., terephthalic acid (TPA), as well as from their esters, such as, e.g., dimethyl terephthalate (DMT), are known to comprise two sequential steps:
(a) a prepolymer production by condensing said diacid or its diester with ethylene glycol; and
(b) production of the aromatic polyester by means of the polycondensation of the above prepolymer, with release of left glycol or water, in the presence of a catalyst, and at an increased temperature and under high vacuum.

One among the most widely used catalysts is antimony trioxide.

The excess of ethylene glycol recovered during the second step, which represents approximately 50% of the fed ethylene glycol, is generally recovered by distillation.

However, this recovered ethylene glycol is impure due to the presence, among others, of diethylene glycol, low molecular weight polyester chains (oligomers), antimony oxide, antimony bound to oligomers, aromatic acids, their esters, several light products from ethylene glycol degradation, such as dioxane, methyl cellosolve, 1,3-methyldioxane, methanol and water.

The above reported steps and the processes for recovering the excess of ethylene glycol are disclosed, e.g., in U.S. patent Nos. 2,778,373, 2,793,235 and 3,311,544.

Although ethylene glycol can be recovered by means of fractional distillation, most residual impurities remain in the bottom of the distillation towers as a granular, waxy mass, which must be removed from the production cycle and disposed of as waste material.

A typical composition of the tower bottom residue, including water and small amounts of sodium or potassium hydroxide added in order to neutralize the acidity of the raw glycol, is as follows:

| | |
|---|---|
| * antimony (as metal): | 0.4-0.6% |
| * sodium (as metal): | 0.6-1% |
| * diethylene glycol: | 15-20% |
| * oligomers: | 50-70% |
| * residual ethylene glycol: | 20-30%. |

Disposing of this residue constitutes a considerably serious problem. In fact, all methods known and proposed hitherto display the drawback that they either release noxious substances (glycols, antimony, and so forth) into the surrounding environment, or they require a so high cost for landfill storage that such a procedure is not acceptable as an industrial solution. Furthermore, the government regulations, more and more sensible to the environmental problem, make such a disposal an unfeasible one.

Also chemical treatments of the tower bottom residues from the production of aromatic polyesters were proposed in the past; however, no one of the processes known from the prior art yielded industrially acceptable results, because all of such treatments are very burdensome and complex as regards the investment and the operations and, furthermore, the obtained raw materials are very difficult to be purified up to the required purity level for the application in the manufacture of fibre-grade polyesters.

The chemical methods proposed by the prior art can be:
(i) methanolysis with recovery of the dimethyl ester of the aromatic diacid and ethylene glycol, as disclosed in U.K. patent Nos. 1,458,486 and 2,041,916 and in U.S. patent Nos. 3,907,868; 4,163,860; 3,776,945 and 3,321,510;
(ii) hydrolysis as disclosed in U.K. patent No. 2,123,403; or,
(iii) alkaline hydrolysis and precipitation of the aromatic diacid, as disclosed in U.S. patent No. 4,013,519 and in Eastern Germany patent No. 96,966.

Furthermore, all of the chemical methods known from the prior art are affected by the problem arising from the need for disposing of sodium, antimony and high-boiling degradation products, which constitute 2-3% by weight of the starting material submitted to treatment.

A purpose of the present invention is of solving the above reminded drawbacks.

More particularly, a purpose of the present invention is of providing a process which makes it possible the excess of ethylene glycol used in the manufacture of aromatic polyesters to be totally recovered, without incuring the above reported drawbacks.

A further purpose of the present invention is of providing an easily and cheaply practiced process which makes it possible all of the raw materials, i.e., ethylene glycol, aromatic diacid or ester thereof, and the catalyst, to be integrally recovered, without any processing residues.

In its general aspect, the present invention makes it possible these and still further purposes to be achieved by submitting to polymerization the tower bottom residue from the fractional distillation of ethylene glycol, possibly diluted with fresh ethylenic glycol, with an aromatic dicarboxy acid, and/or, an ester thereof.

Therefore, the object of the present invention is a method for recovering the tower bottom residues resulting from the distillation of the excess of ethylene glycol from the manufacturing process for aromatic polyesters, consisting of:
(A) feeding the tower bottom residue to an esterification reactor;
(B) feeding, to the same reactor, an aromatic dicarboxy acid or a diester thereof, either alone or, possibly, together with ethylene glycol, in such an amount that the molar ratio of glycol units:aromatic acid or ester thereof, in the resulting mixture, is higher than 1.5;
(C) submitting said mixture to slow heating up to approximately 220°C, with water, or, when a diester is used, the alcohol, being distilled off, in order to form the prepolymer; and
(D) submitting the resulting prepolymer to polymerization, at a temperature higher than 250°C, and under vacuum.

The aromatic poly(ethylene ester) obtained by means of the process according to the present invention is characterized by the presence of metals, in such amounts as to endow the polymer with nucleating properties at crystallization time. In particular, when the aromatic dicarboxy acid fed to the esterification reactor is terephthalic acid, the poly(ethylene terephthalate) obtained by means of the process according to the present invention displays the following properties:

| | |
|---|---|
| -- inherent viscosity: | 0.5-0.7 dl/g; |
| -- melting point: | 210-230°C; |
| -- crystallization temperature from molten polymer: | 180-190°C; |
| -- crystallization temperature from amorphous polymer: | 110-130°C; |
| -- diethylene glycol content: | 5-15% by weight; |
| -- metal content: | at least 0.2% by weight. |

Furthermore, the second-melting poly(ethylene glycol) displays, when analyzed by the Differential Scanning Calorimeter (DSC), endothermic peaks at the temperatures of 200-220°C and, respectively, 220-240°C.

Any esterification reactors, either known or proposed for preparing the polyesters, can be used in the process according to the present invention; preferably used is a stirred batch vertical reactor.

The polymerization can be carried out in the same reactor inside which the esterification is performed, or it can be carried out in a separate reactor, which may also be of the same type, according to techniques known from the prior art.

Any aromatic dicarboxy acids of from 8 to 14 carbon atoms can be fed to the esterification reactor. Of said aromatic dicarboxy acids, up to 50% by mol can be replaced by another, different aromatic dicarboxy acid of from 8 to 14 carbon atoms, and/or up to 20% by mol can be replaced by an aliphatic dicarboxy acid of from 2 to 12 carbon atoms.

Examples of aromatic dicarboxy acids which can be used in the process according to the present invention comprise: terephthalic acid, isophthalic acid, dibenzoic acid, naphthalene dicarboxy acid, including 1,5-; 2,6-; and 2,7-naphthalene dicarboxy acids, 4,4'-diphenylene dicarboxy acid, ethylene-bis-p-benzoic acid, and so forth.

The aliphatic dicarboxy acids, which may possibly be used in order to replace the aromatic acids up to the above reported amounts, include: adipic acid, glutaric acid, sebacic acid, azelaic acid, dodecanedioic acid, 1,4-cyclohexanedicarboxy acid, and so forth.

Terephthalic acid either alone, or mixed with 0.5-15% by mol of isophthalic acid and the dimethyl esters of such acids are preferred in the process according to the present invention.

The addition of ethylene glycol to the tower bottom residue is carried out in order to obtain a reaction mixture in which the molar ratio of glycol units:aromatic acid is higher than 1.5 and preferably is comprised within the range of from 2 to 2.5. However, such an addition may be avoided if the recovery of ethylene glycol by distillation is carried out in such a way as to obtain a tower bottom containing at least 50% by weight of said glycol, based on total mixture. Such a content can be obtained, e.g., by interrupting the distillation cycle at an earlier time. By operating in that way, it is only necessary to add the aromatic dicarboxy acid and/or an ester thereof which, together with the acid already contained in the residue as oligomers, makes it possible the above defined molar ratio to be obtained.

When the present invention is practiced, the bottoms from the distillation tower used in order to distil the poly(ethylene ester) reaction products are first analyzed in order to determine their contents of ethylene glycol and of the acid and/or its ester. Subsequently, and if necessary, pure ethylene glycol is added in order to increase the total molar content of said glycol in the mixture, up to a value of at least 1.5, and preferably from 2 to 2.5 times as large as the content of total dicarboxy acid.

The polymerization of the prepolymer obtained from the step (C) of the process of the present invention, is preferably carried out at a temperature comprised within the range of from 270 to 290°C, and under a reduced pressure, lower than 100 Pa, and which may be as low as 5 Pa.

A further advantageous characteristic of the process according to the present invention is that the addition of catalysts is no longer necessary, because the same alkali metals, such as sodium and/or potassium, and antimony, already present in the tower bottoms, are active as transesterification and respectively polycondensation catalysts. The same holds true for the stabilizers.

The characteristics of the polyesters obtained by means of the process according to the present invention and, in particular, the contents of alkali metals and diethylene glycol and the temperatures of crystallization from the molten material, make it possible the polymer to be classified as inherently nucleated, with a high crystallization rate, and inherently plasticized. Owing to these features, the polyesters obtained by means of the process according to the present invention are particularly suitable for being used as moulding materials, displaying extremely good processability characteristics when used in moulding processes inside low-temperature and high-temperature metal moulds.

The polyesters obtained by means of the process according to the present invention can be admixed with inert reinforcer fillers such as, for example, fiberglass, carbon fibers, asbest fibers, organic fibers of aramidic type and/or non-fibrous inorganic materials, like mica, zeolites, aluminum silicate, graphite, talc, metal oxides and carbonates according to well-known techniques commonly used for such kind of products.

The amount of reinforcer fillers which can be added varies as a function of the desired end characteristics and can generally be comprised within the range of from 2 to 200 parts by weight, and preferably of from 5 to 100 parts by weight, based on 100 parts of polyester by weight.

Furthermore, to the polyester obtained from the process according to the present invention, usual additives and/or auxiliary substances, if necessary, in order to improve its characteristics of oxidative thermal stability and light stability, as well as such other additives as pigments, dyes, flame retardants, mould release agents, other types of thermoplastic resins or rubbers, in order to confer impact strength characteristics, may be added. These additives are generally used in amounts comprised within the range of from 0.1 to 50% by weight.

In order to better understand the present invention and to practice it, in the following some examples are reported in order to illustrate the present invention without limiting it.

In the examples, the following test methods were used for measuring the characteristics of the polymer.

### Inherent viscosity

The inherent viscosity was determined on a polymer solution in ortho-chlorophenol containing 0.5 g/dl of polymer, at 30°C.

### Melting point

The temperature assumed to be the melting point is that temperature value which corresponds to the maximum value of the second endothermic peak as determined on the E.I. Du Pont de Nemours' Differential Scanning Calorimeter (DSC) Model 9900.

### Evaluation ot the crystallization index

On the polymer powder, thermal analyses were carried out by means of a Differential Scanning Calorimeter (DSC) Model 9900, ex E.I. Du Pont de Nemours, in order to evaluate the characteristics thereof. The following parameters were determined: the crystallization temperature from molten material Tc(C), the crystallization temperature from amorphous material Tc(H) and the second melting temperature (SMT) peaks.

The thermograms were obtained with a scanning ramp of 10°C/minute during the heating stage and of 20°C/minute during the cooling stage, always under a blanketing nitrogen atmosphere.

### Amount of diethylene glycol

The amount of diethylene glycol contained in the polymer was determined by submitting the polymer to gas-cromatographic analysis after methanolysis.

### Metal content

The amounts of metals present in the polymer were determined by atomic absorption analysis on a Perkin Elmer spectrometer.

### EXAMPLE 1

To a reactor of 1 litre of capacity, equipped with stirring means, distillation column, reflux condenser, thermoregulator and high vacuum pump, the following materials were charged:
-- 328 g of dimethyl terephthalate, and
-- 300 g of tower bottoms not submitted to complete distillation of ethylene glycol, having the following composition, as weight percent:

| | |
|---|---|
| * ethylene glycol: | 63; |
| * diethylene glycol: | 10; |
| * oligomers: | 18 (as TPA equivalents); |
| * antimony: | 0.2; |
| * sodium: | 0.25; |
| * impurities: | balance to 100. |

Under atmospheric pressure, the mixture was heated up to 245°C within a 2-hour time, with methanol and approximately 20 cc of ethylene glycol being distilled off.

1.2 g of TiO₂ opacifier was added.

The distillation column was put off line and during a 40-minute time, the pressure was gradually decreased down to 10 Pa, with ethylene glycol being distilled off up to the mixture temperature of 292°C. The resulting polymer was extruded at the same temperature of 292°C, under nitrogen pressure, and was cooled in a water bath.

The characteristics of the obtained polyester are reported in the following table.

### EXAMPLE 2

To the same autoclave as of Example 1, the following were charged:
-- 300 g of tower bottoms from ethylene glycol distillation, having the same composition as of Example 1, and
-- 280 g of terephthalic acid.

Under atmospheric pressure, the mixture was heated up to 277°C, with 155 cc of ethylene glycol and water being distilled off, during an approximate time of 2 hours. The pressure was gradually decreased down to a vacuum of 4 Pa and the mixture was heated up to 292°C during 40 minutes. The resulting mixture was extruded at 292°C, under nitrogen pressure, and the obtained polymer was cooled in a water bath.

The characteristics of the resulting polymer are reported in the following table.

**TABLE**

| Characteristics | | Example 1 | Example 2 |
|---|---|---|---|
| - Inherent viscosity (dl/g) | | 0.632 | 0.635 |
| - Melting temperature (°C) | | 227 | 228 |
| - Tc (H) (°C) | | 122 | 121 |
| - Tc (C) (°C) | | 182 | 176 |
| - SMT (H): | 1^{st} peak (°C) | 208 | 218 |
| | 2^{nd} peak (°C) | 229 | 230 |
| - Diethylene glycol content (%) | | 7.5 | 7.6 |
| - Metal content (% by weight) | | 0.4 | 0.4 |

## Claims

1. Method for recovering the tower bottom residues produced by the fractional distillation of the excess of ethylene glycol resulting from the process for manufacturing an aromatic polyester based on such a glycol, characterized in that said method consists in submitting to polymerization said tower bottom residues from fractional distillation of ethylene glycol, possibly diluted with fresh ethylenic glycol, with an aromatic dicarboxy acid, and/or, an ester thereof.

2. Method according to claim 1, characterized in that it consists of:
(A) feeding the tower bottom residue to an esterification reactor;
(B) feeding, to the same reactor, an aromatic dicarboxy acid or a diester thereof, either alone or, possibly, together with ethylene glycol, in such an amount that the molar ratio of glycol units:aromatic acid or ester thereof, in the resulting mixture, is higher than 1.5;
(C) submitting said mixture to slow heating up to approximately 220°C, with water, or, when a diester is used, the alcohol, being distilled off, in order to form the prepolymer; and
(D) submitting the resulting prepolymer to polymerization, at a temperature higher than 250°C, and under vacuum.

3. Method according to claim 1 or 2, in which said aromatic dicarboxy acid contains from 8 to 14 carbon atoms.

4. Method according to any of the preceding claims, in which the aromatic dicarboxy acid is replaced, up to 50% by mol, by another, different aromatic dicarboxy acid of from 8 to 14 carbon atoms, and/or by up to 20% by mol of an aliphatic dicarboxy acid of from 2 to 12 carbon atoms.

5. Method according to any of the preceding claims, in which the aromatic acid or its diester is selected from terephthalic acid either alone or mixed with 0.5-15% by mol of isophthalic acid and the dimethyl ester of such acids.

6. Method according to any of the preceding claims, in which the ethylene glycol is added to the tower bottom residue until a reaction mixture is obtained which contains a molar ratio of glycol units:aromatic acid higher than 1.5 and preferably comprised within the range of from 2 to 2.5.

7. Method according to any of the preceding claims, in which the polymerization of the prepolymer obtained from the step (C), is carried out at a temperature comprised within the range of from 270 to 290°C, and under a reduced pressure, lower than 100 Pa and of up to 5 Pa.

8. Aromatic poly(ethylene ester) obtained by means of the method according to any of the preceding claims.

9. Poly(ethylene terephthalate) obtained by means of the method according to any of the preceding claims from 1 to 7 and having the following properties:
| | |
|---|---|
| -- inherent viscosity: | 0.5-0.7 dl/g; |
| -- melting point: | 210-230°C; |
| -- crystallization temperature from molten polymer: | 180-190°C; |
| -- crystallization temperature from amorphous polymer: | 110-130°C; |
| -- diethylene glycol content: | 5-15% by weight; |
| -- metal content: | at least 0.2% by weight. |

10. Poly(ethylene terephthalate) according to claim 9, characterized in that the second-melting polymer displays, when analyzed by means of the Differential Scanning Calorimeter (DSC), two endothermic peaks, at the temperatures of 200-220°C and, respectively, 220-240°C.
